# EUROPEAN PATENT SPECIFICATION

(11) **EP 4 482 584 B1**
(45) Date of publication and mention of the grant of the patent: **18.11.2026**
(21) Application number: 23708149.2
(22) Date of filing: 20.02.2023
(51) Int. Cl.: A61P 31/14, C07D 487/04

(54) **TRYPTANTHRIN DERIVATIVES WITH THIOSEMICARBAZONE SUBSTITUTION AND USE THEREOF**
TRYPTANTHRIN DERIVATE MIT THIOSEMICARBAZON-SUBSTITUTION UND DEREN VERWENDUNG
DÉRIVÉS TRYPTANTHRINE AVEC SUBSTITUTION DE THIOSEMICARBAZONE ET LEURS UTILISATION

(30) Priority: 22.02.2022 CZ 20220082
(43) Date of publication of application: 01.01.2025
(73) Proprietor: Univerzita Karlova, 116 36 Praha 1 (CZ)
(72) Inventor: KEJIK, Zdenek, 35601 Sokolov (CZ); KAPLANEK, Robert, 10200 Praha 10-Hostivar (CZ); VESELA, Katerina, 78391 Unicov (CZ); SMETANA, Karel, 14100 Praha 4-Zabehlice (CZ); LACINA, Lukas, 14300 Praha 4-Modrany (CZ); MARTASEK, Pavel, 19017 Praha 9-Vinor (CZ); JAKUBEK, Milan, 73532 Rychvald (CZ)
(74) Representative: Jirotkova, Ivana
(86) International application number: PCT/CZ2023/050007
(87) International publication number: WO 2023/160736

(56) References cited:
- EP-A1- 3 421 472
- WO-A2-2004/064759
- TSAI YU-CHI ET AL: "Antiviral Action of Tryptanthrin Isolated from Strobilanthes cusia Leaf against Human Coronavirus NL63", BIOMOLECULES, vol. 10, no. 366, 27 February 2020 (2020-02-27), pages 1 - 17, XP093031417, DOI: 10.3390/biom10030366
- ATASEVER ARSLAN BELKIS ET AL: "The iron(III) and nickel(II) complexes with tetradentate thiosemicarbazones. Synthesis, experimental, theoretical characterization, and antiviral effect against SARS-CoV-2", JOURNAL OF MOLECULAR STRUCTURE, ELSEVIER AMSTERDAM, NL, vol. 1246, 22 July 2021 (2021-07-22), XP086825980, ISSN: 0022-2860, [retrieved on 20210722], DOI: 10.1016/J.MOLSTRUC.2021.131166
- PALABINDELA RAMBABU ET AL: "/T predictions", JOURNAL OF HETEROCYCLIC CHEMISTRY, vol. 59, no. 9, 3 April 2022 (2022-04-03), US, pages 1533 - 1550, XP093031422, ISSN: 0022-152X, Retrieved from the Internet <URL:https://onlinelibrary.wiley.com/doi/full-xml/10.1002/jhet.4488> DOI: 10.1002/jhet.4488
- VESELÁ KATERINA ET AL: "Preparation of Tryptanthrin Derivates Bearing a Thiosemicarbazone Moiety to Inhibit SARS-CoV-2 Replication", DEPARTMENT OF PAEDIATRICS AND INHERITED METABOLIC DISORDERS, FIRST FACULTY OF MEDICINE, CHARLES UNIVERSITY AND GENERAL UNIVERSITY HOSPITAL IN PRAGUE, KE KARLOVU 455/2, 128 08 PRAGUE, CZECH REPUBLIC, 20 January 2023 (2023-01-20), pages 73, XP093039887, Retrieved from the Internet <URL:https://www.mdpi.com/journal/separations> [retrieved on 20230418], DOI: 10.3390/separations10020073

## Description

### Technical Field

The invention relates to tryptanthrin derivatives with thiosemicarbazone substitution of general formula **I** and **II** and to use thereof as inhibitors of virus particle SARS-CoV-2 production.

### Background Art

Tryptanthrins belong to the indoloquinazoline alkaloids. Basic tryptanthrin (6,12-dihydro-6,12-dioxoindolo-(2,1-*b*)-quinazoline) is a yellow compound. Its structural motif contains a quinazoline ring fused to an indole heterocycle with carbonyl groups at positions 6 and 12 (R. Kaur, S.K. Manjal, R.K. Rawal, K. Kumar: Recent synthetic and medicinal perspectives of tryptanthrin. Bioorg. Med. Chem. 25 (2017) 4533-4552; A.M. Tucker, P. Grundt: The chemistry of tryptanthrin and its derivatives. Arkivoc (i) (2012) 546-569). It was first isolated from a *Candida lipolytica* yeast culture and later isolated from a Chinese medicinal plant *Strobilanthes cusia Kuntze (Acanthaceae).* As a potential therapeutic agent, it arouses great interest due to its structural simplicity, the possibility to prepare substitutionally different derivatives, and especially due to its wide spectrum of biological activities. These include antimicrobial activity against various species of *Trichophyton, Microsporum* and *Epidermophyron* genera, *Leishmania donovani, Trypanosoma brucei* and *Plasmodium falciparum, Mycobacterium tuberculosis,* anti-inflammatory activity (inhibition of cyclooxygenase-2 and reduction of nitric oxide synthase expression), antiviral or antifungal activity. Antitumor activity of tryptanthrin *in vitro* was observed in a number of cancer cell lines, including leukemia U937, breast tumor MCF-7, glioma U251, colon tumor SW620 and lung tumor H5229 (R. Kaur, S.K. Manjal, R.K. Rawal, K. Kumar: Recent synthetic and medicinal perspectives of tryptanthrin. Bioorg. Med. Chem. 25 (2017) 4533-4552; G.M. Shankar, J. Antony, R.J. Anto: Quercetin and Tryptanthrin: Two Broad Spectrum Anticancer Agents for Future Chemotherapeutic Interventions. Enzymes 37 (2015) 43-72; J. Kawakami, N. Matsushima, Y. Ogawa, H. Kakinami, A. Nakane, H. Kitahara, M. Nagaki, S. Ito: Antibacterial and Antifungal Activities of Tryptanthrin Derivatives. Trans. Mat. Res. Soc. Japan 36 (2011) 603-606; J.M. Hwang, T. Oh, T. Kaneko, A.M. Upton, S.G. Franzblau, Z. Ma, S.N. Cho, P. Kim: Design, Synthesis, and Structure-Activity Relationship Studies of Tryptanthrins As Antitubercular Agents. J. Nat. Prod. 76 (2013) 354-367; Y. Hao, J. Guo, Z. Wang, Y. Liu, Y. Li, D. Ma, Q. Wang: Discovery of Tryptanthrins as Novel Antiviral and Anti-Phytopathogenic-Fungus Agents. J. Agric. Food Chem. 68 (2020) 5586-5595; Y. Jahng: Progress in the studies on tryptanthrin, an alkaloid of history. Arch. Pharm. Res. 36 (2013) 517-535).

In addition, predictions have recently begun to appear that the tryptanthrin structural motif could exhibit a therapeutic effect against coronaviruses, e.g., SARS-CoV-2 (Y.C. Tsai, C.L. Lee, H.R. Yen, YS Chang, Y.P. Lin, S.H. Huang, C.W. Lin: Antiviral Action of Tryptanthrin Isolated from Strobilanthes cusia Leaf against Human Coronavirus NL63. Biomolecules 10 (2020) 366; J.S. Mani, J.B. Johnson, J.C. Steel, D.A. Broszczak, P.M. Neilsen, K.B. Walsh, M. Naiker: Natural product-derived phytochemicals as potential agents against coronaviruses: A review. Virus Research 284 (2020) 197989; Y. Hao, J. Guo, Z. Wang, Y. Liu, Y. Li, D. Ma, Q. Wang: Discovery of Tryptanthrins as Novel Antiviral and Anti -Phytopathogenic- Fungus Agents. J. Agric. Food Chem. 68 (2020) 5586-5595; B.C. Fielding, C.S.M.B. Filho, N.S.M. Ismail, D. Pergentino de Sousa: Alkaloids: Therapeutic Potential against Human Coronaviruses. Molecules 258 (2020) 5496; R.R. Narkhede, A.V. Pise, R.S. Cheke, S.D. Shinde: Recognition of Natural Products as Potential Inhibitors of COVID-19 Main Protease (Mpro): In-Silico Evidences. Nat. Prod. Biopersp. 10 (2020) 297-306; S.N. Sahu, B. Mishra, R. Sahu, S.K. Pattanayak: Molecular dynamics simulation perception study of the binding affinity performance for main protease of SARS-CoV-2, J. Biomol. Struct. Dynamo (2020) DOI: 10.1080/07391102.2020.1850362).

SARS-CoV-2 represents a type of highly pathogenic human coronavirus, causing the disease COVID-19. The pandemic of this disease has a major impact on the public health system and the economy of states. Effective treatment for the disease COVID-19 is still limited and its availability, especially in the so-called third world countries, is very limited. While the vast majority of patients with COVID-19 have a good prognosis, a number of patients have a severe course of the disease that requires hospitalization, optionally respiratory support. Some of these have resulted in death. The main problem is that this phenomenon can often occur without symptoms or with mild symptoms (mild fever, cough or muscle aches). However, in the later stages of the disease or even in the recovery process, acute respiratory distress syndrome (ARDS) and multi-organ failure can occur within a short period of time, which can have a fatal impact on the patient. One of the main causes of this phenomenon is generally considered to be the cytokine storm that was found in critical patients with COVID-19 (K. Smetana, Jr., J. Brábek: Role of interleukin-6 in lung complications in patients with COVID-19: Therapeutic implications. In Vivo 34 (2020) 1589-1592; M. Soy, G. Keser, P. Atagündüz, F. Tabak, I. Atagündüz, S. Kayhan: Cytokine storm in COVID-19: pathogenesis and overview of anti-inflammatory agents used in treatment. Clin. Rheumatol. 39 (2020) 2085-2094; Q. Ye, B. Wang, J. Mao: The pathogenesis and treatment of the 'Cytokine Storm' in COVID-19. J. Infect. 80 (2020) 607-613). Based on this, it can be legitimately assumed that effective suppression of the cytokine storm could very substantially mitigate this serious disease, thereby effectively saving a number of lives and significantly mitigating further national-economic damage in the outbreak of the next expected wave of the epidemic.

For these reasons, drugs to suppress the cytokine storm in patients with COVID-19 are being intensively studied (K. Smetana, Jr., D. Rösel, J. Brábek: Raloxifene and bazedoxifene could be promising candidates for preventing the COVID-19 related cytokines storm, ARDS and mortality. In Vivo 34 (2020) 3027-3028; J. Brábek, M. Jakubek, F. Vellieux, J. Novotný, M. Kolá 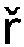 , L. Lacina, P. Szabo, K. Strnadová, D. Rösel, B. Dvořánková, K. Smetana, Jr.: Interleukin-6: Molecule in the intersection of cancer, aging and COVID-19. Int. J. Mol. Sci. 21 (2020) 7937; W. Zhang, Y. Zhao, F. Zhang, Q. Wang, T. Li, Z. Liu, J. Wang, Y. Qin, X. Zhang, X. Yan, X. Zeng, S. Zhang: The use of anti -inflammatory drugs in the treatment of people with severe coronavirus disease 2019 (COVID-19): The Perspectives of clinical immunologists from China. Clin. Immunol. 214 (2020) 108393; M. Dalamaga, I. Karampela, CS Mantzoros: Commentary: Could iron chelators prove to be useful as an adjunct to COVID-19 Treatment Regimens? Metabolism 108 (2020) 154260; M. Edeas, J. Saleh, C. Peyssonnaux: Iron: Innocent bystander or vicious culprit in COVID-19 pathogenesis? Int. J. Infect. Dis. 97 (2020) 303-305). Tested agents include pegylated and non-pegylated interferons, corticosteroids, intravenous immunoglobulin, interleukin 1 and 6 antagonists, tumor necrosis factor α blockers, interferon-α/β antagonists, ulinastatin, oxidized phospholipids, and sphingosine-1-phosphate receptor 1 antagonists. Studies show that these agents can contribute to mitigating the course of the disease in certain stages of the disease, however, their effectiveness is still substantially insufficient.

Some studies indicate that it is a disease associated with a substantial increase in the level of iron in the blood (M. Soy, G. Keser, P. Atagündüz, F. Tabak, I. Atagündüz, S. Kayhan: Cytokine storm in COVID-19: pathogenesis and overview of anti-inflammatory agents used in treatment. Clin. Rheumatol. 39 (2020) 2085-2094; Q. Ye, B. Wang, J. Mao: The pathogenesis and treatment of the 'Cytokine Storm' in COVID-19. J. Infect. 80 (2020) 607-613). This fact suggests that a promising therapy could be based on the administration of chelators for ferrous and ferric ions of natural origin, or on already approved drugs with a chelating effect. It is known that such substances show, thanks to their chelating effects, immunomodulating and antiviral effects, especially against RNA viruses, e.g., SARS-CoV-2. It is widely believed that these agents could attenuate ARDS and moderate the course of the disease through a variety of mechanisms (inhibition of viral replication, reduction of iron availability, upregulation of B cells, increase in the titer of neutralizing antiviral antibodies, inhibition of endothelial inflammation, and prevention of pulmonary fibrosis and lung loss by reducing the accumulation of iron in the lungs.

Patent application WO 2004/064759 describes tryptanthrin analogs of formula 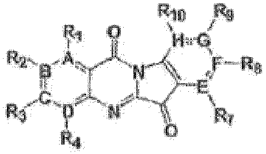 and their medical use to enhance immune response against viruses, such as corona viruses.

Patent application EP 3421472 discloses iron(III) and nickel(II) complexes with tetradentate thiosemicarbazones, including an indoloquinazoline moiety, and their antiviral effect against SARS-CoV-2.

One of the suitable groups for binding transition metal ions, especially ferric and ferrous, are thiosemicarbazones. Combining a tryptanthrin pharmacophore and the thiosemicarbazone chelating group will result in derivatives that have the desired properties for potential use as inhibitors of SARS-CoV-2 viral particle production. Thus, these substances combine a suitable structural motif for targeting the SARS-CoV-2 protease with effectively chelating ferric and ferrous ions. The use of tryptanthrin derivatives with thiosemicarbazone substitution for the inhibition of SARS-CoV-2 with the aim of applying these substances in the therapy of the disease COVID-19 is the subject of this patent application.

### Disclosure of Invention

The invention provides tryptanthrin derivatives with thiosemicarbazone substitution of the general formula I, 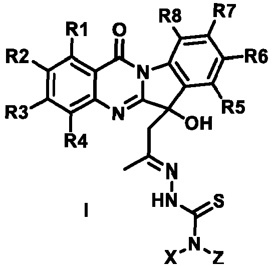 where R1-R8 are independently H, OH, alkyl with 1 to 6 carbon atoms, C(CH₃)₃, allyl, propargyl, benzyl, phenyl, F, Cl, Br, I, CH₂OH, O(alkyl), CF₃, OCF₃, CN, COOH, COO(alkyl),
CONH₂, CONH(alkyl), NO₂, N(alkyl)₂, NH(alkyl), NHCO(alkyl), where the alkyl has 1 to 6 carbon atoms,
or R1-R2 or R2-R3 or R3-R4 or R5-R6 or R6-R7 or R7-R8 is -CH=CH-CH=CH-, i.e., a fused benzene ring,
X and Z are independently H, alkyl of 1 to 6 carbon atoms, benzyl, phenyl.

The invention further provides tryptanthrin derivatives with thiosemicarbazone substitution of general formula **II,** 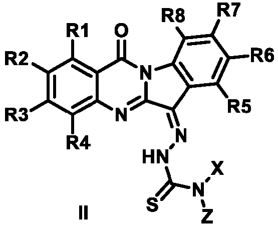 where R1-R8, X and Z are as defined above.

The substances of general formulae I and **II** show a high affinity (represented by the binding energy) for the papain-like SARS-CoV-2 protease (PL^{pro} ), a key enzyme for the replication of the SARS-CoV-2 virus.

Therefore, the invention further provides the use of these substances for the production of a drug for treatment using the inhibition of the production of viral particles SARS-CoV-2, specifically for the treatment of coronavirus diseases.

In addition, we have observed that these substances show a significant selectivity for ferric and ferrous ions. In addition, the obtained values of the binding constants prove that their affinity is high enough to manifest itself also in the biological environment and have a significant therapeutic impact. The obtained results could not be predicted and had to be obtained experimentally. Synthetic receptors based on tryptanthrin derivatives for transition metal ions have already been described. However, they showed selectivity and affinity for aluminum or copper ions without significant interaction with ferrous and ferric ions (J. Kawakami, K. Kikuchi, K. Chiba, N. Matsushima, A. Yamaya, S. Ito, M. Nagaki, H. Kitahara: 2-Aminotryptanthrin derivative with pyrene as a FRET- based fluorescent chemosensor for Al3+. Anal. Sci. 25 (2009) 1385-1386; J. Kawakami, Y. Kinami, M. Takahashi, S. Ito: 2-Hydroxytryptanthrin and 1-Formyl-2-hydroxytryptanthrin as Fluorescent Metal-ion Sensors and Near-infrared Fluorescent Labeling Reagents. Trans. Mat. Res. Soc. Japan 43 (2018) 109-112; Y. Liang, X. Wang, H. Fang, N. Han, C. Wang, Z. Xiao, A. Zhu, J. Liu: A Highly Selective and Sensitive Colorimetric Probe for Cu 2+ Determination in Aqueous Media Based on Derivative of Tryptanthrin. Anal. Sci. 34 (2018) 1111-1115); or non-specific chelation of a number of transition metal ions (J. Kawakami, M. Sasagawa, S. Ito: 2-Hydroxy-1-((2-(pyridin-2-yl)-hydrazono)methyl)tryptanthrin as and Fluorescent Chemosensor for Metal Ions. Trans. Mat. Res. Soc. Japan 43 (2018) 209-212). Also, their structural motif was significantly different and has no connection with the subject of this patent application.

In addition, we have observed that the tested substances show strong affinity and selectivity for RNA over DNA. Since SARS-CoV-2 is an RNA virus, this phenomenon increases their therapeutic effectiveness. The interaction of tryptanthrin derivatives with DNA has already been described, however, these are substances with a significantly different structure, which are not the subject of this patent application (P. Langer, J.T. Anders, K. Weisz, J. Jähnchen: Efficient Synthesis of 2-Alkylidene-3-iminoindoles, Indolo[1,2-b]isoquinolin-5-ones, δ-Carbolines, and Indirubines by Domino and Sequential Reactions of Functionalized Nitriles. Chem. Eur. J. 9 (2003) 3951-3964; Y.N. Zhong, Y. Zhang, Y.Q. Gu, S.Y. Wu, W.Y. She, M.X. Tan: Novel Fell and Coll Complexes of Natural Products Tryptanthrin: Synthesis and Binding with G-Quadruplex DNA. Bioinorg. Chem. Appl. (2016) 5075847; RJ Terryn 3rd, H.W. German, T.M. Kummerer, R.R. Sinden, J.C. Baum, M.J. Novak: Novel computational study on π-stacking to understand mechanistic interactions of Tryptanthrin analogues with DNA. Toxicol. Mech. Methods 24 (2014) 73-79; G.S. Chen, B.V. Bhagwat, P.Y. Liao, H.T. Chen, S.B. Lin, J.W. Chern: Specific stabilization of DNA triple helices by indolo[2,1-b]quinazolin-6,12-dione derivatives. Bioorg. Med. Chem. Lett. 17 (2007) 1769-1772; A. Popov, A. Klimovich, O. Styshova, T. Moskovkina, A. Shchekotikhin, N. Grammatikova, L. Dezhenkova, D. Kaluzhny, P. Deriabin, A. Gerasimenko, A. Udovenko, V. Stonik: Design, synthesis and biomedical evaluation of mostotrin, a new water soluble tryptanthrin derivative. Int. J. Mol. Med. 46 (2020) 1335-1346; L.F. Zhao, Y.C. Liu, Q.P. Qin, W.Z. Ya, H.C. Duan: Tryptanthrin Sulfonate: Crystal Structure, Cytotoxicity and DNA Binding Studies. Adv. Mater. Res. 554-556 (2012) 1694-1699; P.P. Bandekar, K.A. Roopnarine, V.J. Parekh, T.R. Mitchell, M.J. Novak, R.R. Sinden: Antimicrobial activity of tryptanthrins in Escherichia coli. J. Med. Chem. 53 (2010) 3558-3565). Furthermore, their interaction with RNA has not been described for any one of them.

In addition to the above, we have also observed *in vitro* in the Vero cell model that these substances are potent inhibitors of viral mRNA production and therefore of viral replication.

### Brief Description of Drawings

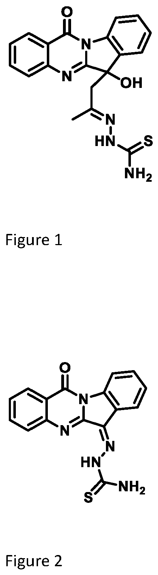

Figure 1 shows the structure of tryptanthrin derivative 1 (PAA-TSC).
Figure 2 shows the structure of tryptanthrin derivative 5 (T8H-TSC).

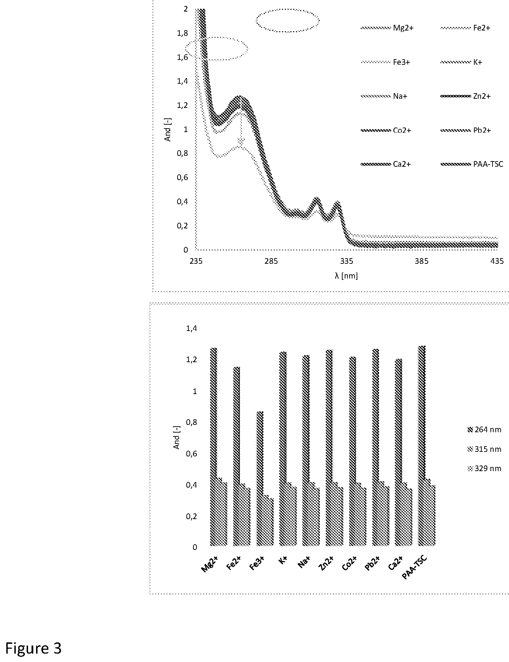

Figure 3 shows the selectivity of tryptanthrin derivative 1 for Fe²⁺/Fe³⁺ ions using UV-Vis spectroscopy. The figure depicts UV/Vis spectra of receptor 1 (PAA-TSC) (100 µM) in the presence and absence of metal ions (5000 µM), including the bar expression in absorption maxima.

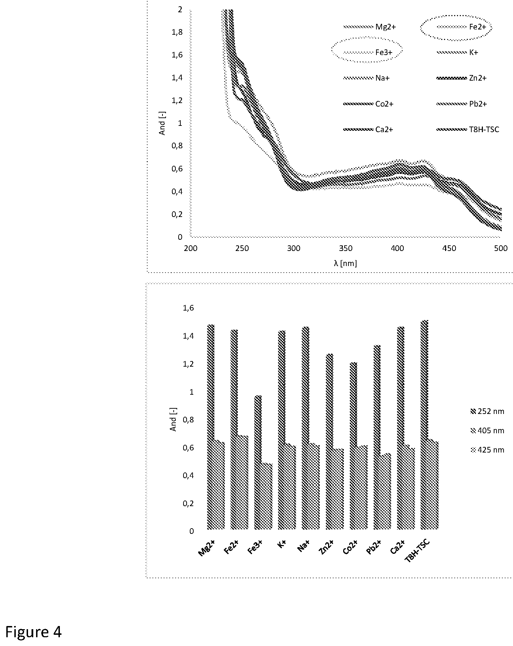

Figure 4 shows the selectivity of tryptanthrin derivative 5 for Fe²⁺/Fe³⁺ ions by UV-Vis spectroscopy. The figure depicts UV/Vis spectra of receptor 5 (T8H-TSC) (100 µM) in the presence and absence of metal ions (5000 µM), including a column expression in absorption maxima.

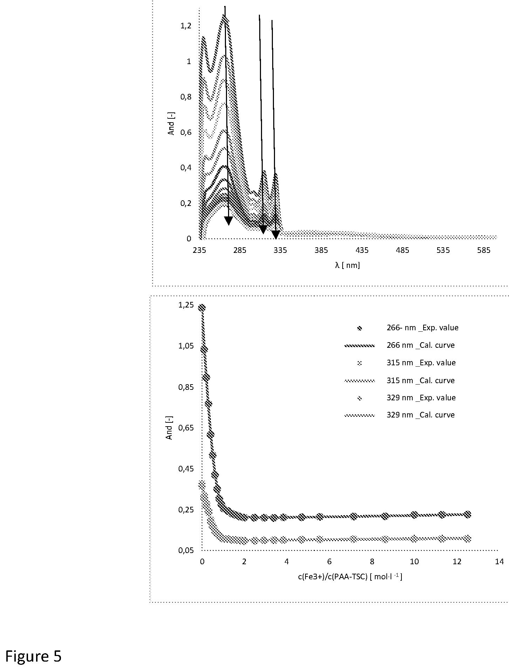

Figure 5 shows the affinity of tryptanthrin derivative 1 for Fe³⁺ ions by UV-Vis spectroscopy. The figure depicts titration (top) and titration curves (bottom) of receptor 1 (PAA-TSC) with Fe³⁺ ion. Titration curves were recorded at the absorption maxima of receptor 1 (PAA-TSC). The graph on the left shows the values of added metal ion equivalents.

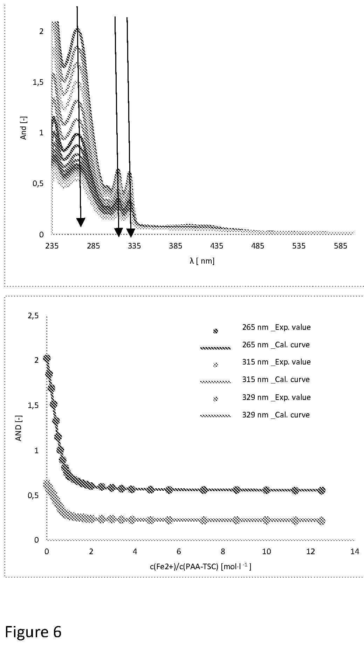

Figure 6 shows the affinity of tryptanthrin derivative 1 for Fe²⁺ ions by UV-Vis spectroscopy. The figure depicts titration (top) and titration curves (bottom) of receptor 1 (PAA-TSC) with Fe²⁺ ion. Titration curves were recorded at the absorption maxima of receptor 1 (PAA-TSC). The graph on the left shows the values of added metal ion equivalents.

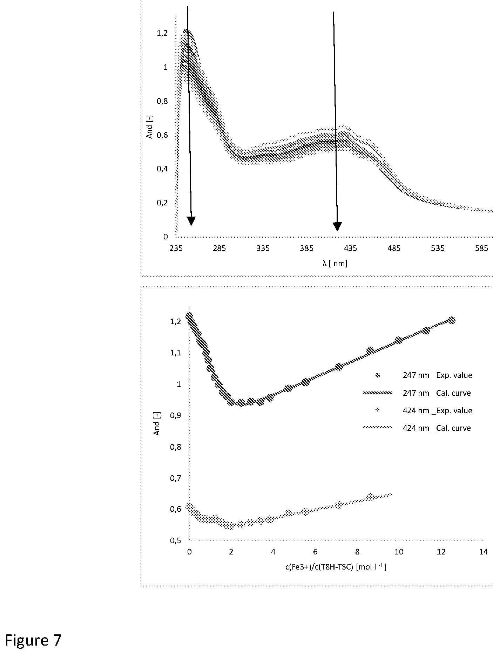

Figure 7 shows the affinity of the tryptanthrin derivative 5 for Fe³⁺ ions by UV-Vis spectroscopy. The figure depicts titration (top) and titration curves (bottom) of receptor 5 (T8H-TSC) with Fe³⁺ ion. Titration curves were recorded at the absorption maxima of receptor 5 (T8H-TSC). The graph on the left shows the values of added metal ion equivalents.

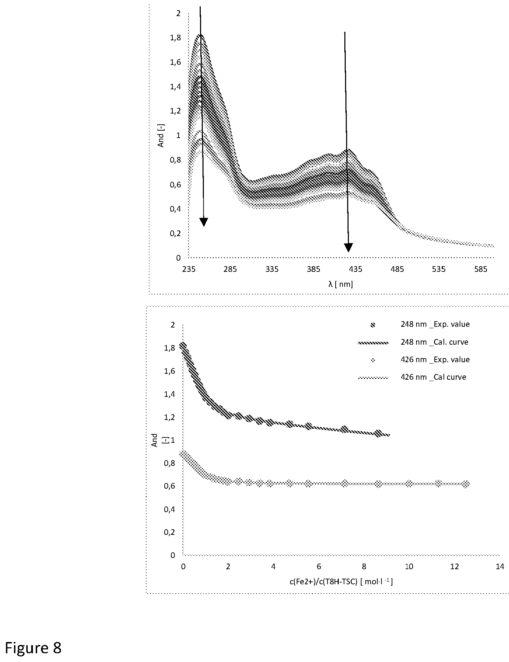

Figure 8 shows the affinity of the tryptanthrin derivative 5 for Fe²⁺ ions by UV-Vis spectroscopy. The figure depicts titration (top) and titration curves (bottom) of receptor 5 (T8H-TSC) with Fe²⁺ ion. Titration curves were recorded at the absorption maxima of receptor 5 (T8H-TSC). The graph on the left shows the values of added metal ion equivalents.

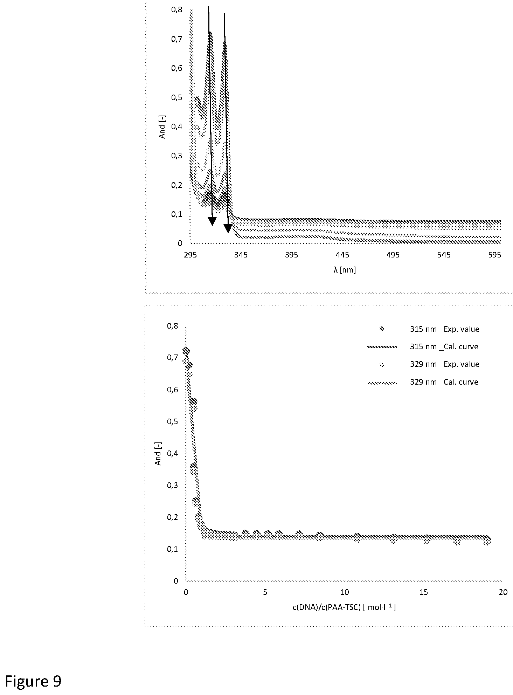

Figure 9 shows the affinity of tryptanthrin derivative 1 for DNA by UV-Vis spectroscopy. The figure depicts titration (top) and titration curves (bottom) of receptor 1 (PAA-TSC) with DNA. Titration curves were recorded at the absorption maxima of receptor 1 (PAA-TSC). The graph on the left shows the values of DNA equivalents added.

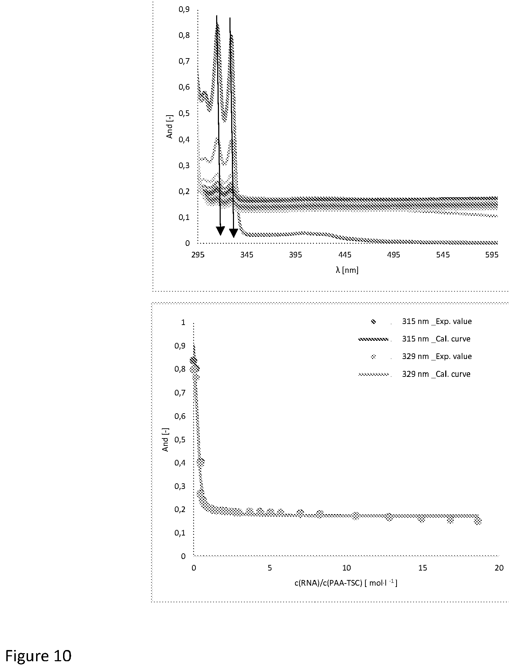

Figure 10 shows the affinity of tryptanthrin derivative 1 for RNA by UV-Vis spectroscopy. The figure depicts titration (top) and titration curves (bottom) of receptor 1 (PAA-TSC) with RNA. Titration curves were recorded at the absorption maxima of receptor 1 (PAA-TSC). The graph on the left shows the values of RNA equivalents added.

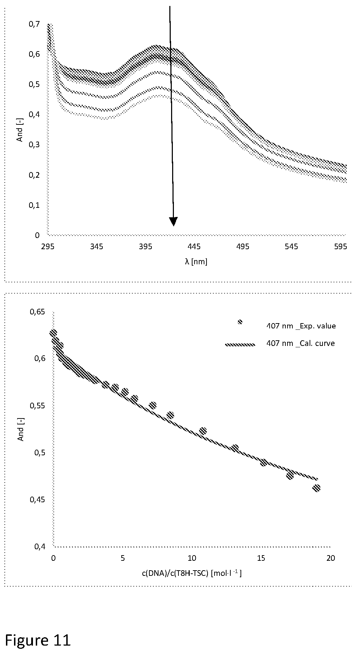

Figure 11 shows the affinity of tryptanthrin derivative 5 for DNA by UV-Vis spectroscopy. The figure depicts titration (top) and titration curves (bottom) of receptor 5 (T8H-TSC) with DNA. Titration curves were recorded at the absorption maxima of receptor 5 (T8H-TSC). The graph on the left shows the values of DNA equivalents added.

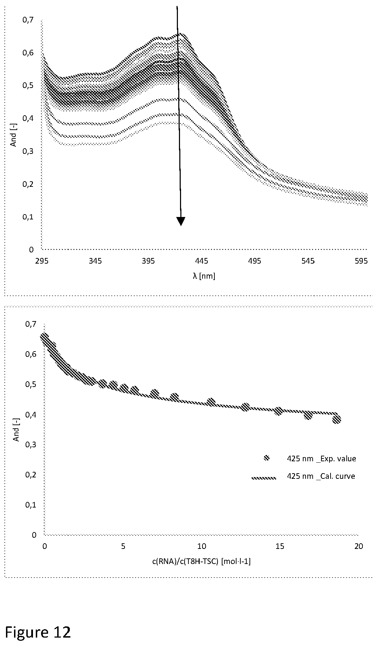

Figure 12 shows the affinity of the tryptanthrin derivative 5 for RNA by UV-Vis spectroscopy. The figure depicts titration (top) and titration curves (bottom) of receptor 5 (T8H-TSC) with RNA. Titration curves were recorded at the absorption maxima of receptor 5 (T8H-TSC). The graph on the left shows the values of RNA equivalents added.

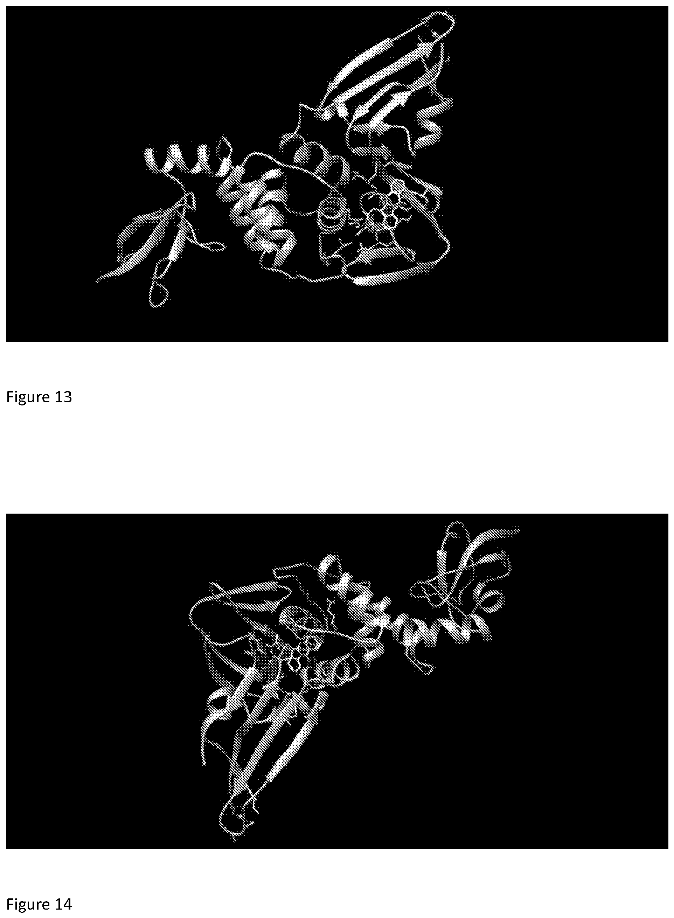

Figure 13 shows the interaction of tryptanthrin derivative 1 with PL^{pro} using molecular docking. The figure depicts an interaction model of 1 (PAA-TSC) with PL^{pro}.
Figure 14 shows the interaction of tryptanthrin derivative 5 with PL^{pro} using molecular docking. The figure depicts an interaction model of 5 (T8H-TSC) with PL^{pro}.

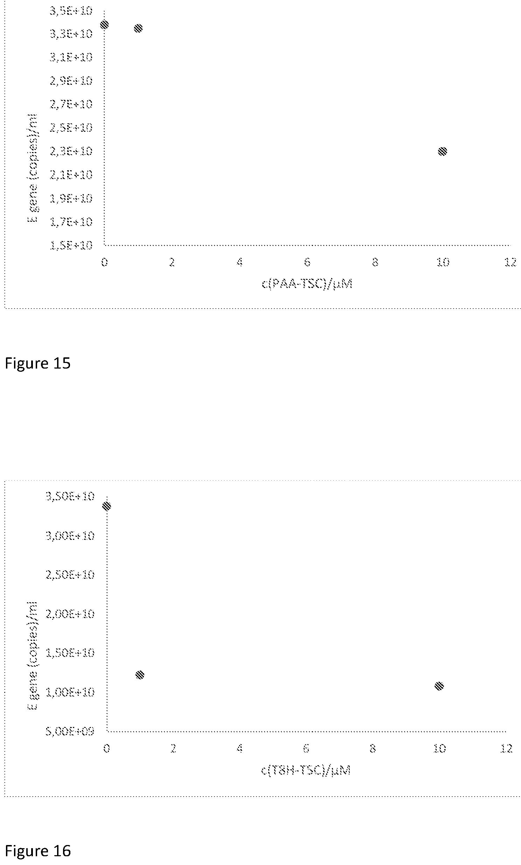

Figure 15 shows inhibition of virus particle production by tryptanthrin derivative 1 (PAA-TSC). The figure depicts effect of concentration of 1 (PAA-TSC) on CoV-2 RNA production in Vero *in vitro* model.
Figure 16 shows inhibition of viral particle production by tryptanthrin derivative 5 (T8H-TSC). The figure depicts effect of concentration of 5 (T8H-TSC) on CoV-2 RNA production in the Vero *in vitro* model.

### Examples

The preparation and properties of tryptanthrin derivatives with thiosemicarbazone substitution are illustrated by, but not limited to, the following examples.

Example 1. Preparation of tryptanthrin derivative 1 (PAA-TSC), falling under the general formula **I.**

Phaitanthrin A (123 mg; 0.4 mmol) and thiosemicarbazide (146 mg; 1.6 mmol) were dissolved in methanol (10 mL) and acetic acid (0.1 mL) was added. The reaction mixture was stirred at 60°C for 12 h. After cooling, the reaction mixture was diluted with water (40 mL), the solid product was filtered off on a frit, washed with water (20 mL) and dried. 120 mg (79%) of compound 1 (PAA-TSC) was obtained. The structure of the derivative is shown in Table 1 and Figure 1. ¹H NMR (DMSO-*d₆*): (2 diastereoisomers): 1.80 (1.87) (s, 3H); 3.35 (m, 2H); 5.92 (s, 1H); 6.53 (s, 1H); 7.39 (t, J = 7.7 Hz, 1H); 7.51 (t, J = 7.7 Hz, 1H); 7.63 (m, 2H); 7.80 (d, J = 8.2 Hz, 1H); 7.89 (m, 2H); 8.29 (8.46) (d, J = 8.0 Hz, 1H); 8.41 (d, J = 8.0 Hz, 1H); 9.79 (10.45) s, 1H). ¹ C NMR (DMSO-*d₆*) (2 diastereoisomers): 17.88; 45.98; 75.83 (76.44); 115.97 (116.26); 121.17 (121.49); 124.34; 126.51 (126.36); 126.69; 127.52 (127.77); 129.67 (130.24); 134.11 (132.89); 134.92 (134.84); 138.44 (138.30); 146.95 (146.77); 150.52 (148.67); 158.74 (158.90); 160.71 (160.09); 178.22 (178.44).

Example 2. Preparation of tryptanthrin derivative **2** (PAA8Cl-TSC), falling under the general formula **I.**

8-Chloro-phaitanthrin A (136 mg; 0.4 mmol) and thiosemicarbazide (146 mg; 1.6 mmol) were dissolved in methanol (10 mL) and acetic acid (0.1 mL) was added. The reaction mixture was stirred at 60°C for 12 h. After cooling, the reaction mixture was diluted with water (40 mL), the solid product was filtered off on a frit, washed with water (20 mL) and dried. 149 mg (90%) of 2 (PAA8Cl-TSC) was obtained. The structure of the derivative is shown in Table 1. ¹H NMR (DMSO-*d₆*): 1.90 (m, 3H); 3.35 (m, 2H); 5.88 (s, 1H); 7.60 (m, 1H); 7.90 (m, 2H); 8.01 (m, 2H); 8.20 (m, 1H); 8.53 (m, 1H); 8.99 (s, 1H).

Example 3. Preparation of tryptanthrin derivative 3 (PAA2Cl-TSC), falling under the general formula **I.**

2-Chloro-phaitanthrin A (136 mg; 0.4 mmol) and thiosemicarbazide (146 mg; 1.6 mmol) were dissolved in methanol (10 mL) and acetic acid (0.1 mL) was added. The reaction mixture was stirred at 60°C for 12 h. After cooling, the reaction mixture was diluted with water (40 mL), the solid product was filtered off on a frit, washed with water (20 mL) and dried. 124 mg (75%) of 3 (PAA2Cl-TSC) was obtained. The structure of the derivative is shown in Table 1. ¹H NMR (DMSO-*d₆*): 1.88 (m, 3H); 3.30 (m, 2H); 6.01 (s, 1H); 7.51 (m, 1H); 7.94 (m, 2H); 8.01 (m, 2H); 8.26 (m, 1H); 8.50 (m, 1H).

Example 4. Preparation of tryptanthrin derivative **4** (PAA-Me₂ TSC), falling under the general formula **I.**

Phaitanthrin A (123 mg; 0.4 mmol) and 4,4-dimethyl-3-thiosemicarbazide (190 mg; 1.6 mmol) were dissolved in methanol (9 mL) and acetic acid (1 mL) was added. The reaction mixture was stirred at 60°C for 12 h. After cooling, the reaction mixture was diluted with water (40 mL), the solid product was filtered off on a frit, washed with water (20 mL) and dried. 143 mg (88%) of **4** (PAA-Me₂TSC) was obtained. The structure of the derivative is shown in Table 1. ¹ H NMR (DMSO-*d₆*): 1.84 (s, 3H); 3.35 (m, 5H); 3.50 (s, 3H); 5.90 (s, 1H); 6.54 (s, 1H); 7.42 (m, 1H); 7.50 (m, 1H); 7.63 (m, 2H); 7.80 (d, J = 8.1 Hz, 1H); 7.91 (m, 2H); 8.35 (m, 1H).

Example 5. Preparation of tryptanthrin derivative 5 (T8H-TSC), falling under the general formula **II.**

Tryptanthrin (87 mg; 0.35 mmol) and thiosemicarbazide (91 mg; 1 mmol) were mixed in ethanol (15 mL) and acetic acid (0.15 mL) was added. The reaction mixture was stirred at 75°C for 48 h. After cooling, the reaction mixture was diluted with water (150 mL), the solid product was filtered off on a frit, washed with water (100 mL) and dried. 106 mg (94%) of 5 (T8H-TSC) was obtained. The structure of the derivative is shown in Table 1 and Figure 2. ¹H NMR (DMSO-*d₆*): 7.46 (t, J = 7.6 Hz, 1H); 7.60 (t, J = 7.8 Hz, 1H); 7.69 (t, J = 7.6 Hz, 1H); 7.78 (d, J = 8.1 Hz, 1H); 7.96 (d, J = 7.8 Hz, 1H); 8.04 (d, J = 7.6 Hz, 1H); 8.32 (d, J = 7.9 Hz, 1H); 8.39 (d, J = 8.1 Hz, 1H); 8.85 (s, 1H); 9.15 (s, 1H); 13.00 (s, 1H). ¹³C NMR (DMSO-ds): 116.34; 121.15; 121.68; 123.14; 126.47; 126.82; 127.75; 128.66; 130.85; 131.06; 135.10; 139.39; 145.77; 157.78; 178.76.

Example 6. Preparation of tryptanthrin derivative 6 (T8OMe-TSC), falling under the general formula **II.**

8-Methoxy-tryptanthrin (97 mg; 0.35 mmol) and thiosemicarbazide (91 mg; 1 mmol) were mixed in ethanol (15 mL) and acetic acid (0.15 mL) was added. The reaction mixture was stirred at 75°C for 12 h. After cooling, the reaction mixture was diluted with water (150 mL), the solid product was filtered off on a frit, washed with water (100 mL) and dried. 113 mg (92%) of 6 (T8OMe-TSC) was obtained. The structure of the derivative is shown in Table 1. ¹H NMR (DMSO-*d₆*): 3.86 (s, 3H); 7.14 (d, J = 8.6 Hz, 1H); 7.69 (m, 2H); 7.78 (d, J = 8.0 Hz, 1H); 7.94 (t, J = 7.4 Hz, 1H); 8.27 (d, J = 8.8 Hz, 1H); 8.31 (d, J = 7.8 Hz, 1H); 8.91 (s, 1H); 9.19 (s, 1H); 12.90 (s, 1H).

Example 7. Preparation of tryptanthrin derivative **7** (T8OTFM-TSC), falling under the general formula **II.**

8-Trifluoromethoxy-tryptanthrin (116 mg; 0.35 mmol) and thiosemicarbazide (91 mg; 1 mmol) were mixed in ethanol (15 mL) and acetic acid (0.15 mL) was added. The reaction mixture was stirred at 75°C for 48 h. After cooling, the reaction mixture was diluted with water (150 mL), the solid product was filtered off on a frit, washed with water (100 mL) and dried. 131 mg (92%) of 7 (T8OTFM-TSC) was obtained. The structure of the derivative is shown in Table 1. ¹ H NMR (DMSO-*d₆*): 7.62 (m, 1H); 7.72 (d, J = 7.5 Hz, 1H); 7.82 (d, J = 8.0 Hz, 1H); 7.98 (m, 1H); 8.11 (s, 1H); 8.34 (t, J = 8.4 Hz, 1H); 8.48 (d, J = 8.8 Hz, 1H); 9.12 (s, 1H); 9.26 (s, 1H); 12.85 (s, 1H).

Example 8. Preparation of tryptanthrin derivative **8** (T8F-TSC), falling under the general formula **II.**

8-Fluoro-tryptanthrin (93 mg; 0.35 mmol) and thiosemicarbazide (91 mg; 1 mmol) were mixed in ethanol (15 mL) and acetic acid (0.15 mL) was added. The reaction mixture was stirred at 75°C for 24 h. After cooling, the reaction mixture was diluted with water (150 mL), the solid product was filtered off on a frit, washed with water (100 mL) and dried. 105 mg (89%) of **8** (T8F-TSC) was obtained. The structure of the derivative is shown in Table 1. ¹ H NMR (DMSO-*d₆*): 7.45 (m, 1H); 7.71 (t, J = 7.5 Hz, 1H); 7.80 (m, 1H); 7.90 (m, 1H); 7.95 (m, 1H); 8.33 (m, 1H); 8.40 (m, 1H); 8.95 (s, 1H); 9.22 (s, 1H); 12.85 (s, 1H).

Example 9. Preparation of tryptanthrin derivative **9** (T8Cl-TSC), falling under the general formula **II.**

8-Chlorotryptanthrin (99 mg; 0.35 mmol) and thiosemicarbazide (91 mg; 1 mmol) were mixed in ethanol (15 mL) and acetic acid (0.15 mL) was added. The reaction mixture was stirred at 75°C for 48 h. After cooling, the reaction mixture was diluted with water (150 mL), the solid product was filtered off on a frit, washed with water (100 mL) and dried. 107 mg (86%) of **9** (T8Cl-TSC) was obtained. The structure of the derivative is shown in Table 1. ¹H NMR (DMSO-*d₆*): 7.68 (m, 2H); 7.80 (m, 1H); 7.96 (m, 1H); 8.16 (s, 1H); 8.35 (m, 2H); 8.99 (s, 1H); 9.37 (s, 1H); 12.82 (s, 1H).

Example 10. Preparation of tryptanthrin derivative **10** (T8Br-TSC), falling under the general formula **II.**

8-Bromo-tryptanthrin (115 mg; 0.35 mmol) and thiosemicarbazide (91 mg; 1 mmol) were mixed in ethanol (15 mL) and acetic acid (0.15 mL) was added. The reaction mixture was stirred at 75°C for 48 h. After cooling, the reaction mixture was diluted with water (150 mL), the solid product was filtered off on a frit, washed with water (100 mL) and dried. 120 mg (86%) of **10** (T8Br-TSC) was obtained. The structure of the derivative is shown in Table 1. ¹ H NMR (DMSO-*d₆*): 7.71 (t, J = 7.5 Hz, 1H); 7.78 (m, 2H); 7.96 (t, J = 7.6 Hz, 1H); 8.32 (m, 3H); 9.00 (s, 1H); 9.34 (s, 1H); 12.81 (s, 1H).

Example 11. Preparation of tryptanthrin derivative **11** (T2Cl-TSC), falling under the general formula **II.**

2-Chloro-tryptanthrin (99 mg; 0.35 mmol) and thiosemicarbazide (91 mg; 1 mmol) were mixed in ethanol (15 mL) and acetic acid (0.15 mL) was added. The reaction mixture was stirred at 75°C for 12 h. After cooling, the reaction mixture was diluted with water (150 mL), the solid product was filtered off on a frit, washed with water (100 mL) and dried. 109 mg (87%) of **11** (T2Cl-TSC) was obtained. The structure of the derivative is shown in Table 1. ¹H NMR (DMSO-*d₆*): 7.50 (m, 1H); 7.90 (m, 2H); 8.04 (m, 2H); 8.29 (m, 1H); 8.48 (m, 1H); 8.98 (s, 1H); 9.83 (s, 1H); 12.01 (s, 1H).

Example 12. Preparation of tryptanthrin derivative **12** (NT8H-TSC), falling under the general formula **II.**

Naphthotryptanthrin (benzo[*g*]indolo[2,1-*b*]quinazoline-6,14-dione; 104 mg; 0.35 mmol) and thiosemicarbazide (91 mg; 1 mmol) were mixed in ethanol (12 mL) and added acetic acid (3 ml). The reaction mixture was stirred at 75°C for 48 h. After cooling, the reaction mixture was diluted with water (150 mL), the solid product was filtered off on a frit, washed with water (100 mL) and dried. 109 mg (84%) of **12** (NT8H-TSC) was obtained. The structure of the derivative is shown in Table 1. ¹ H NMR (DMSO-*d₆*): 7.50 (m, 1H); 7.76 (m, 1H); 7.92 (m, 4H); 8.30 (m, 1H); 8.49 (m, 1H); 8.95 (s, 1H); 9.79 (s, 1H); 11.89 (s, 1H).

Example 13. Preparation of tryptanthrin derivative 13 (T8H-PhTSC), falling under the general formula **II.**

Tryptanthrin (87 mg; 0.35 mmol) and 4-phenyl-3-thiosemicarbazide (167 mg; 1 mmol) were mixed in ethanol (7 mL) and acetic acid (7 mL). The reaction mixture was stirred at 75°C for 48 h. After cooling, the reaction mixture was diluted with water (150 mL), the solid product was filtered off on a frit, washed with water (100 mL) and dried. 120 mg (86%) of 13 (T8H-PhTSC) was obtained. The structure of the derivative is shown in Table 1. ¹ H NMR (DMSO-*d₆*): 7.48 (t, J = 7.8 Hz, 1H); 7.70 (m, 4H); 7.82 (m, 2H); 8.01 (m, 4H); 8.35 (d, J = 7.8 Hz, 1H); 8.42 (m, 1H); 9.95 (s, 1H); 12.88 (s, 1H).

Example 14. Preparation of tryptanthrin derivative **14** (T8H-Me₂ TSC), falling under the general formula **II.**

Tryptanthrin (123 mg; 0.35 mmol) and 4,4-dimethyl-3-thiosemicarbazide (119 mg; 1 mmol) were mixed in acetic acid (7 mL). The reaction mixture was stirred at 75°C for 12 h. After cooling, the reaction mixture was diluted with water (150 mL), the solid product was filtered off on a frit, washed with water (100 mL) and dried. 98 mg (90%) of **14** (T8H-Me₂ TSC) was obtained. The structure of the derivative is shown in Table 1. ¹ H NMR (DMSO-*d₆*): 3.31 (s, 3H); 3.55 (s, 3H); 7.49 (t, J = 7.5 Hz, 1H); 7.64 (t, J = 7.8 Hz, 1H); 7.72 (t, J = 7.5 Hz, 1H); 7.84 (d, J = 8.1 Hz, 1H); 7.88 (d, J = 7.6 Hz, 1H); 7.96 (m, 1H); 8.36 (d, J = 7.9 Hz, 1H); 8.46 (d, J = 8.0 Hz, 1H).

**Table 1. Structures of tryptanthrin derivatives shown in Examples 1-14**

| | |
|---|---|
| **Derivative (general formula I)** | 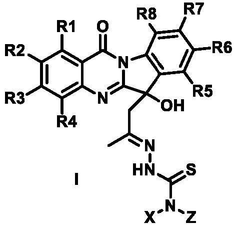 |
| **1** (PAA-TSC) | R1= R2 = R3 = R4 = R5 = R6 = R7 = R8 = H; X = Z = H |
| **2** (PAA8Cl-TSC) | R1 = R2 = R3 = R4 = R5 = R7 = R8 = H, R6 = Cl; X = Z = H |
| **3** (PAA2Cl-TSC) | R1 = R3 = R4 = R5 = R6 = R7 = R8 = H, R2 = Cl; X = Z = H |
| **4** (PAA-Me ₂TSC) | R1= R2 = R3 = R4 = R5 = R6 = R7 = R8 = H; X = Z = CH₃ |
| **Derivative (general formula II)** | 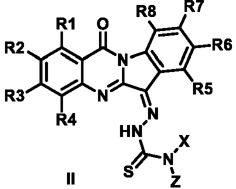 |
| **5** (T8H-TSC) | R1= R2 = R3 = R4 = R5 = R6 = R7 = R8 = H; X = Z = H |
| **6** (T8OMe-TSC) | R1= R2 = R3 = R4 = R5 = R7 = R8 = H, R6 = OCH₃; X = Z = H |
| **7** (T8HOTFM-TSC) | R1= R2 = R3 = R4 = R5 = R7 = R8 = H, R6 = OCF₃; X = Z = H |
| **8** (T8F-TSC) | R1= R2 = R3 = R4 = R5 = R7 = R8 = H, R6 = F; X =Z = H |
| **9** (T8Cl-TSC) | R1 = R2 = R3 = R4 = R5 = R7 = R8 = H, R6 = Cl; X = Z = H |
| **10** (T8Br-TSC) | R1= R2 = R3 = R4 = R5 = R7 = R8 = H, R6 = Br; X = Z = H |
| **11** (T2Cl-TSC) | R1 = R3 = R4 = R5 = R6 = R7 = R8 = H, R2 = Cl; X = Z = H |
| **12** (NT8H-TSC) | R1= R4 = R5 = R6 = R7 = R8 = H, R2-R3 = CH=CH-CH=CH; X = Z = H |
| **13** (T8H-PhTSC) | R1 = R2 = R3 = R4 = R5 = R6 = R7 = R8 = H; x = H, Z = Ph |
| **14** (T8H-Me ₂ TSC) | R1= R2 = R3 = R4 = R5 = R6 = R7 = R8 = H; X = Z = H |

### Example 15. Interaction of tryptanthrin derivatives with transition metal ions

First, the interaction of tryptanthrin derivatives **1** and **5** with metal ions was studied using UV/Vis spectroscopy. The receptor concentration was 100 µM and the ion concentration was 5000 µM. After addition of the metal ions, significant absorbance changes were observed only in the case of ferrous and ferric ions. The absorbance of tryptanthrin derivatives 1 and 5 in the absence and presence of metal ions is shown in Figure 3 and Figure 4, resp.

### Example 16. Determination of tryptanthrin derivatives binding constant with for Fe²⁺ and Fe³⁺ ions

The association of tryptanthrin derivatives with ferrous and ferric ions was studied by UV/Vis spectroscopy in aqueous solution (water/DMSO, 99:1, v/v). Since the solvent significantly affects the binding constants, all titrations were performed in the same medium and the ratio of DMSO to water was kept constant. Association constants (K) were calculated from absorbance changes (*ΔA*) in the spectral maximum of the derivative and in the spectral maximum of its complexes with Fe^{2+/3+} ions by regression analysis. The concentration of tryptanthrin derivatives was 100 µM. The concentration of Fe²⁺ and Fe³⁺ ions fluctuated in the range of 0-5 mM. UV/Vis spectra were measured with a Shimadzu spectrophotometer in the range 220-900 nm with a step of 1 nm in a 1 cm plastic cuvette at a scanning speed of 300 nm·min⁻¹. The effect of different concentrations of Fe ions on the absorbance of substance 1 (PAA-TSC) is shown in Figures 5 and 6; that for substance 5 (T8H-TSC) in Figures 7 and 8. The calculated association constants and stoichiometry of the complexes are shown in Table 2.

**Table 2. Calculated values of association constants K, stoichiometry of the mentioned complexes.**

| **Tryptanthrin derivative** | **Ion** | **Log (*K*)** | **Stoichiometry (ion:ligand)** |
|---|---|---|---|
| PAA-TSC **(1)** | Fe²⁺ | 1.5 | 1:1 |
| | | 13.7 | 1:2 |
| | Fe³⁺ | 6.0 | 1:1 |
| | | 10.2 | 1:2 |
| T8H-TSC (5) | Fe²⁺ | 4.9 | 1:1 |
| | | 7.1 | 2:1 |
| | Fe³⁺ | 12.2 | 1:1 |
| | | 17.4 | 2:1 |

Example 17. Study of the interaction of tryptanthrin derivatives with DNA and RNA.

The interaction between receptors and DNA/RNA was studied using UV/Vis spectrometry. The desired amount was taken from the stock solutions of receptors 1 or 5 and diluted in phosphate buffer (the pH of the buffer was adjusted to pH = 7.00) to a concentration of 10⁻⁴ M. A solution of DNA from salmon sperm was prepared from 75 mg of this DNA and 15 ml of phosphate buffer. The RNA solution was prepared by dissolving 35 mg of RNA in 15 ml of phosphate buffer. Data were collected with a Shimadzu spectrophotometer in the range of 200-800 nm with an accuracy of 1 nm in a 1 cm plastic cuvette. Then association constants (K) were calculated from changes in absorbance (*ΔA*) by regression analysis using the *Letagrop Spefo 2005* software. The effect of different concentrations of DNA/RNA on the absorbance of 1 (PAA-TSC) is shown in Figures 9 and 10. The effect of different concentrations of DNA/RNA on the absorbance of 5 (T8H-TSC) is shown in Figures 11 and 12. The calculated association constants and stoichiometry of the resulting of the complexes are shown in Table 3.

**Table 3. Calculated values of association constants K including the determination of the stoichiometry of the mentioned complexes.**

| **Tryptanthrin derivative** | **NK** | **Log (*K*)** | **Stoichiometry (NK:ligand)** |
|---|---|---|---|
| PAA-TSC **(1)** | DNA | 7.4 | 1:1 |
| | RNA | 16.5 | 1:1 |
| | | 8.7 | 2:1 |
| T8H-TSC **(5)** | DNA | 6.0 | 1:2 |
| | RNA | 7.3 | 1:2 |

### Example 18. Study of interaction of PL^{pro} with tryptanthrin derivatives using computational methods

Docking of Tryptanthrin Derivatives to the PL^{pro} Model. The three-dimensional structure of PL^{pro} was obtained from the Protein Data Bank database with PDB ID. 3D structural models of tryptanthrin derivatives were built using MolView (https://molview.org). Subsequently, the corresponding mol files containing the 3D coordinates were converted to .pdb format using Open Babel. For further preparation of the coordinate files and for docking, software from the AutoDock Vina suite was used. A similar approach based on the crystal structure of human PL^{pro} was used to dock tryptanthrin derivatives to the PL^{pro} molecule. An orthorhombic box of size 52 x 58 x 50 A³ including the PL^{pro} molecule was used for the calculations.

The values of affinities and binding energies obtained by docking tryptanthrin derivatives are summarized in Table 4. Figures 13 and 14 show the visualization of the interaction of tryptanthrin derivatives **1** and **5,** resp., with PL^{pro}.

**Table 4. Binding energies of tryptanthrin derivatives 1 and 5 for PL^{pro}**

| | Binding energy (kcal/mol) | Inhibition constant |
|---|---|---|
| PAA-TSC **(1)** | -5.34 | 121.07 |
| T8H-TSC **(5)** | -6.57 | 15.37 |

### Example 19. Antiviral effects of tryptanthrin derivatives 1 (PAA-TSC) and 5 (T8H-TSC)

The cells in question (Vero cell line from African green monkey kidney; 1.5 x 10⁵ cells per well) were infected with a SARS-CoV-2 isolate provided by the Bioconservation Section of Techonin. The infectious inoculum contained 2.38 x 10⁷ copies of the SARS -CoV-2 E-gene. After 1 h inoculation, cells were supplemented with 1 ml of Dulbecco 's modified Eagle's medium containing 2% fetal bovine serum (2% FBS-DMEM) and increasing concentrations (0-10 µM) of tryptanthrin derivatives **1** (PAA-TSC) and **5** (T8H-TSC), resp. After 24 and 48 hours of incubation, the growth of SARS-CoV-2 was characterized in an aliquot of the culture supernatant by one-step RT-qPCR.

Viral RNA was isolated from 200 µl culture supernatant using magnetic beads. SARS-CoV-2 RNA was quantified by amplifying the E-gene of SARS-CoV-2 (Generi Biotech) using the SensiFast Probe One-Step Kit (BioLine) and Light Cycler 480 II (Roche) using absolute quantification and calibration curve. The primers and probes used are listed in Table 5. The effect of tryptanthrin derivatives **1** (PAA-TSC) and **5** (T8H-TSC) on viral RNA production is shown in Figures 15 and 16, resp.

**Table 5. Primers and probes used:**

| Name | 5'-3' sequence | Concentration in reaction |
|---|---|---|
| E_Sarbeco_F1 | ACAGGTACGTTAATATAGTTAATAGCGT | 400 nM |
| E_Sarbeco_R2 | ATATTGCAGCAGTACGCACACA | 400 nM |
| E_Sarbeco_P1 | FAM-ACACTAGCCATCCTTACTGCGCTTCG-BHQ1 | 200 nM |

### Industrial Applicability

The invention relates to tryptanthrin derivatives with thiosemicarbazone substitution of the general formulae I and **II.** The given substances can be used for the preparation of medicine to suppress coronavirus infections, especially SARS-CoV-2 infection.

## Claims

1. Tryptanthrin derivatives with thiosemicarbazone substitution of general formulae **I** and **II,** 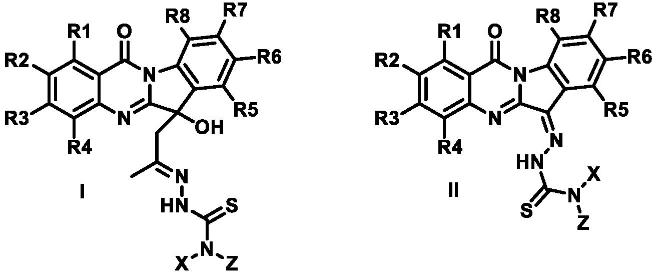
where R1-R8 are independently H, OH, alkyl with 1 to 6 carbon atoms, C(CH₃)₃, allyl, propargyl, benzyl, phenyl, F, Cl, Br, I, CH₂OH, O(alkyl), CF₃, OCF₃, CN, COOH, COO(alkyl), CONH₂, CONH(alkyl), NO₂, N(alkyl)₂, NH(alkyl), NHCO(alkyl), where the alkyl has 1 to 6 carbon atoms,
or R1-R2 or R2-R3 or R3-R4 or R5-R6 or R6-R7 or R7-R8 is -CH=CH-CH=CH-, i.e., a fused benzene ring,
X and Z are independently H, alkyl of 1 to 6 carbon atoms, benzyl or phenyl.

2. Use of tryptanthrin derivatives with thiosemicarbazone substitution of the general formulae **I** and **II** according to claim 1 for the production of a drug.

3. Tryptanthrin derivatives with thiosemicarbazone substitution of general formulae I and II according to claim 1 for use as a drug for the treatment of coronavirus diseases.

## Patentansprüche

1. Tryptanthrinderivate mit Thiosemicarbazon-Substitution der allgemeinen Formeln I und **II,** 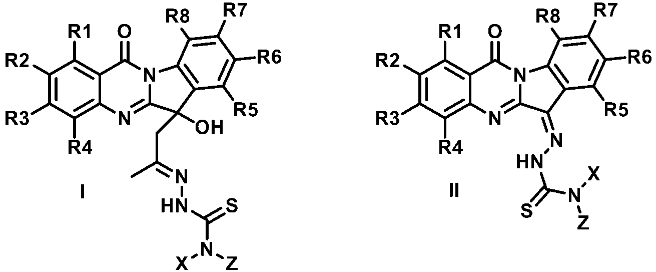
wobei R1-R8 unabhängig voneinander H, OH, Alkyl mit 1 bis 6 Kohlenstoffatomen, C(CH₃) _{3,} Allyl, Propargyl , Benzyl, Phenyl, F, Cl, Br, I, CH ₂ OH, O(Alkyl), CF₃, OCF₃, CN, COOH, COO(Alkyl), CONH _{2,} CONH(Alkyl), NO_{2,} N(Alkyl)_{2,} NH(Alkyl), NHCO(Alkyl) sind, wobei das Alkyl 1 bis 6 Kohlenstoffatome hat,
oder R1-R2 oder R2-R3 oder R3-R4 oder R5-R6 oder R6-R7 oder R7-R8 ist -CH=CH-CH=CH-, d.h. ein kondensierter Benzolring,
X und Z sind unabhängig voneinander H, Alkyl mit 1 bis 6 Kohlenstoffatomen, Benzyl oder Phenyl.

2. Verwendung von Tryptanthrinderivaten mit Thiosemicarbazon-Substitution der allgemeinen Formeln **I** und **II** nach Anspruch 1 zur Herstellung eines Arzneimittels.

3. Tryptanthrinderivate mit Thiosemicarbazon-Substitution der allgemeinen Formeln I und II nach Anspruch 1 zur Verwendung als Arzneimittel zur Behandlung von Coronavirus-Erkrankungen.

## Revendications

1. Dérivés de tryptanthrine avec substitution thiosemicarbazone de formules générales **I** et **II,** 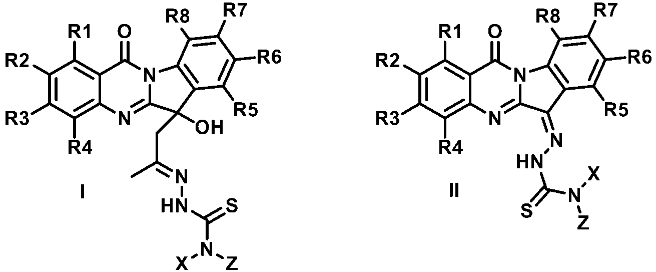
où R1-R8 sont indépendamment H, OH, alkyle avec 1 à 6 atomes de carbone, C(CH₃)₃, allyle, propargyle, benzyle, phényle, F, Cl, Br, I, CH ₂ OH, O(alkyle), CF₃, OCF₃, CN, COOH, COO(alkyle), CONH₂, CONH(alkyle), NO_{2,} N(alkyle)_{2,} NH(alkyle), NHCO(alkyle), où l'alkyle a 1 à 6 atomes de carbone,
ou R1-R2 ou R2-R3 ou R3-R4 ou R5-R6 ou R6-R7 ou R7-R8 est -CH=CH-CH=CH-, c'est-à-dire un cycle benzénique fusionné,
X et Z sont indépendamment H, alkyle de 1 à 6 atomes de carbone, benzyle ou phényle.

2. Utilisation de dérivés de tryptanthrine avec substitution thiosemicarbazone des formules générales **I** et **II** selon la revendication 1 pour la production d'un médicament.

3. Dérivés de tryptanthrine avec substitution thiosemicarbazone de formules générales I et II selon la revendication 1 pour une utilisation comme médicament pour le traitement des maladies à coronavirus.
